# EUROPÄISCHE PATENTSCHRIFT

(11) **EP 2 548 524 B1**
(45) Veröffentlichungstag und Bekanntmachung des Hinweises auf die Patenterteilung: **09.04.2014**
(21) Anmeldenummer: 12177289.1
(22) Anmeldetag: 20.07.2012
(51) Int. Cl.: A61B 17/28, A61B 19/00

(54) **Zange**
Clipper
Pince

(30) Priorität: 22.07.2011 DE 102011052091
(43) Veröffentlichungstag der Anmeldung: 23.01.2013
(73) Patentinhaber: Karl Storz GmbH & Co. KG, 78532 Tuttlingen (DE)
(72) Erfinder: Bogdoll, Robert, 78532 Tuttlingen (DE)
(74) Vertreter: Weiss, Peter

(56) Entgegenhaltungen:
- WO-A1-99/55241
- DE-A1- 4 402 677
- DE-A1-102005 004 085
- DE-U1- 20 200 668
- DE-U1-202010 010 843
- US-A- 5 690 673
- US-A1- 2007 112 377

## Beschreibung

### TECHNISCHES GEBIET

Die Erfindung betrifft eine Zange nach den Merkmalen des Oberbegriffs des Anspruchs 1 sowie eine Verwendung einer Spülpistole und einen Aufsatz für eine Spülpistole.

### STAND DER TECHNIK

Aus dem Stand der Technik sind eine Vielzahl von Zangen bekannt. Die Reinigung dieser Instrumente ist relativ aufwendig und bereitet in der Praxis immer wieder Schwierigkeiten.

In diesem Zusammenhang wird auf die US 2007/112377 A1 hingewiesen, welche eine Laparoskopie- Zange gemäß dem Oberbegriff des Anspruchs 1 offenbart. Weiter wird auf die DE 44 02 677 A1 verwiesen, welche ein Verfahren und Vorrichtung zum Spülen einer Rohrschaftzange offenbart. Daneben wird auf die DE 202 00 668 U1 hingewiesen, welche ein chirurgisches Instrument aufzeigt, bei dem ein Fluidkanal vorhanden ist, welcher einen Fluideinlass an seinem proximalen Ende und eine Fluidströmung in distaler Richtung ermöglicht. Zuletzt wird auf die WO 99/55241A1 hingewiesen, welche eine Branche für ärztliche und zahnärztliche Verwendung.

### BESCHREIBUNG DER ERFINDUNG

Aufgabe der Erfindung ist es, eine Zange zu schaffen, die besser gereinigt werden kann, und kostengünstig herstellbar ist, sowie geeignete Reinigungsgeräte bereit zu stellen.

Zur Lösung der Aufgabe führen die Merkmale des kennzeichnenden Teiles des Anspruchs 1, und die Merkmale des Anspruchs 8.

Eine Zange, insbesondere eine Zange für chirurgische Zwecke umfasst bevorzugt eine erste Zangenhälfte und eine zweite Zangenhälfte, wobei die Zangenhälften über zumindest ein Gelenk, welche auch als Rollen bezeichnet werden können miteinander verbunden sind. Für das einfachere Verständnis werden die Rollen im Folgenden als Gelenke bezeichnet. Zweckmässigerweise weist zumindest eine der Zangenhälften zumindest einen Spülkanal auf. Dadurch ergibt sich der Vorteil, dass die Zange besser gereinigt werden kann.

Im Folgenden sind mit Zangen aller Arten von Zangen, insbesondere für chirurgische Zwecke gemein. Beispielhaft aber nicht abschliessend kann die Zange als einfache Zange, übersetzte Zangen, Doppelrolleninstrument, Hohlmeisezange, Knabberzange od. dgl. ausgebildet sein.

In typischen Ausführungsbeispielen weist der Spülkanal einen Durchmesser von 0,5 mm bis 8 mm, bevorzugt 1 mm bis 3 mm, besonders bevorzugt 1,5 mm auf. Dadurch ergibt sich der Vorteil, dass der Spülkanal relativ klein ist und kein oder nur wenig Schmutz bei der Behandlung durch den Spülkanal in den Behandlungsbereich gelangt. Ein weiterer Vorteil des geringen Durchmessers des Spülkanals ist, dass das Bauteil durch eine kleine Öffnung möglichst wenig geschwächt wird.

Vorzugsweise ist der Spülkanal von "aussen" mit einer Senkung versehen. Dabei ist mit "aussen" die Seite des Spülkanals gemeint, die ohne Demontage und/oder Betätigen der Zange frei zugänglich ist. Besonders bevorzugt weist die Senkung einen Durchmesser von ca. 2 mm auf. Dadurch ergibt sich der Vorteil, dass Vorrichtungen zur Reinigung leicht positioniert und/oder angesetzt werden können.

In typischen Ausführungsbespielen ist der Spülkanal so angeordnet, dass er eine Aussenseite bzw. Oberfläche der Zangenhälfte mit einer Innenfläche eines Funktionselements des Gelenks verbindet. Dadurch ergibt sich der Vorteil, dass die Innenflächen des Gelenks bzw. der Funktionselemente des Gelenks besser gereinigt werden können.

In typischen Ausführungsbeispielen besteht ein Gelenk bevorzugt aus einem männliches Funktionselement, das in ein weibliches Funktionselement greift. Noch bevorzugter sind die Funktionselemente über eine Schraube verbunden.

In typischen Ausführungsbeispielen weisen die Funktionselemente des Gelenks ein Spiel zueinander auf. Dadurch ergibt sich der Vorteil, dass Spülflüssigkeit, die durch den Spülkanal eingebracht wird, und Verschmutzungen, die durch die Spülflüssigkeit ausgewaschen werden, besser aus dem Gelenk herauslaufen können.

In typischen Ausführungsbeispielen ist der Spülkanal so angeordnet, dass er ein weibliches Funktionselement des Gelenks mit einer Oberfläche der Zangenhälfte verbindet. Dadurch ergibt sich der Vorteil, dass die Vertiefung in dem weiblichen Funktionselement des Gelenks, in der leicht Verschmutzungen hängen bleiben, besser gereinigt werden kann.

In typischen Ausführungsbeispielen weist der Spülkanal eine Spüldüse auf. Bevorzugt ist die Spüldüse geeignet zur Verbindung mit einer Spülpistole. Bevorzugt ist die Spüldüse einstückig mit dem Spülkanal ausgebildet. Dadurch ergibt sich der Vorteil, dass eine herkömmliche Spülpistole so an die Spüldüse des Spülkanals angesetzt werden kann, dass eine Spülung möglich ist.

In typischen Ausführungsbeispielen ist der Spülkanal geeignet eine Spüldüse aufzunehmen. Zweckmässigerweise wird dazu eine geeignete Spüldüse in den Spülkanal gesteckt. Dadurch ergibt sich der Vorteil, dass das Spülen sehr einfach möglich ist.

In typischen Ausführungsbeispielen umfasst der Spülkanal ein Gewinde, geeignet eine Spüldüse aufzunehmen. Bevorzugt weist die Spüldüse ein entsprechendes Aussengewinde zum Einschrauben in das Gewinde des Spülkanals auf. Dadurch ergibt sich der Vorteil, dass eine gute Verbindung zwischen Spüldüse und Spülkanal erzeugt werden kann.

In typischen Ausführungsbeispielen weist zumindest eine der Zangenhälften eine Ausnehmung auf. Bevorzugt ist die Ausnehmung zwischen einem Maulteil und einer Branche der Zange angeordnet.

Besonders bevorzugt ist die Ausnehmung an einer Innenseite der Zangenhälfte angeordnet, so dass die Zangenhälfte in einem unbelasteten Zustand der Zange zwischen dem Maulteil und der Branche zumindest teilweise voneinander beabstandet sind. Dadurch ergibt sich der Vorteil, dass beim Spülen der Zange in der Spülmaschine eine bessere Reinigung möglich ist, da die voneinander beabstandeten Flächen gereinigt werden können. Ein weiterer Vorteil ist, dass bei Zangen mit den bereits beschriebenen Spülkanälen die Spülflüssigkeit und Verschmutzung, die beim Spülen des Spülkanals aus dem Gelenk herausgespült werden noch besser ablaufen können.

In typischen Ausführungen sind die Ausnehmungen oval, rund und oder mit einem Radius versehen. Dadurch dass keine scharfen Kanten vorliegen ergibt sich der Vorteil, dass keine erhöhte Kerbspannung, die zu Brüchen führen kann, auftritt.

In typischen Ausführungsbeispielen sind die Ausnehmungen symmetrisch gegenüberliegend in beide Zangenhälften eingebracht. Dadurch ergibt sich der Vorteil, dass beide Zangenhälften noch weiter voneinander beabstandet werde. Auch ist vorteilhaft, dass die Zangenhälften ähnlich ausgebildet sind und so die gleichen Eigenschaften bspw. bezüglich Gewicht und Stabilität aufweisen.

In typischen Ausführungsbeispielen umfasst die Zange eine Mehrzahl von Gelenken. Dadurch ergibt sich der Vorteil, dass die Zange eine übersetzte Zange ist. Bevorzugt ist bei einer Zange mit einer Mehrzahl von Gelenken zumindest eine Ausnehmung an einem Zwischenstück, das die Gelenke verbindet, vorgesehen. Dadurch ergibt sich der Vorteil, dass die relativ langen Zwischenstücke in unbelastetem Zustand voneinander beabstandet sind und dadurch in Reinigungsgeräten oder Spülmaschinen besser gereinigt werden können.

Gesondert wird Schutz beansprucht für eine Verwendung einer Spülpistole zum Spülen eines Spülkanals einer Zange nach den beschriebenen Merkmalen. Bevorzugt ist die Spülpistole so ausgebildet, dass sie mit dem Spülkanal in der Zange in Wirkverbindung gebracht werden kann, so dass aus der Spülpistole Spülflüssigkeit in den Spülkanal eingebracht werden kann.

Gesondert wird ein Aufsatz für eine Spülpistole offenbart zum Spülen eines Spülkanals einer Zange mit den beschriebenen Merkmalen. Bevorzugt ist der Aufsatz geeignet in den Spülkanal einzugreifen. Bevorzugt weist der Aufsatz einen Adapter, geeignet zur Verbindung mit herkömmlichen Spülpistolen auf. Dadurch ergibt sich der Vorteil, dass die erfindungsgemässe Zange mit herkömmlichen Spülpistolen auf einfache Art und Weise gereinigt werden kann.

### Kurze Beschreibung der Zeichnung

Nachfolgend wird die Erfindung anhand der beiliegenden Figur kurz beschrieben:

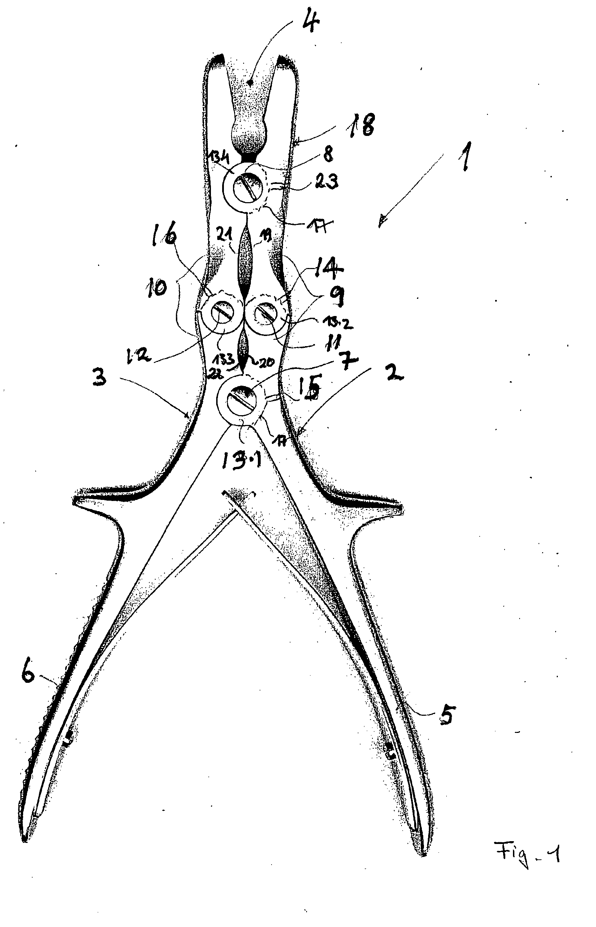

Figur 1 zeigt eine schematische Darstellung einer Draufsicht einer chirurgischen übersetzten Zange.

### Ausführungsbeispiel

Figur 1 zeigt eine Zange 1, insbesondere für chirurgische Zwecke. Die Zange 1 umfasst eine erste Zangenhälfte 2 und eine zweite Zangenhälfte 3. Die beiden Zangenhälften 2 und 3 bilden ein Zangemaul 4 aus. Jede der Zangenhälften 2 und 3 weist eine Branche 5 und 6 auf.

Bei der Zange 1 handelt es sich um eine sogenannte übersetzte Zange. Deshalb weist die Zange 1 ein erstes Gelenk 7 und ein zweites Gelenk 8 auf.

Ein weiterer Begriff für Gelenke ist Rolle. Diese werden im folgenden gleichbedeutend verwendet.

Die Gelenke 7 und 8 sind jeweils über ein Zwischenstück 9 und ein Zwischenstück 10 der ersten Zangenhälfte 2 und der zweiten Zangenhälfte 3 verbunden. Jedes der Zwischenstücke 9 und 10 umfasst ein Knickgelenk 11 bzw. 12.

Alle Gelenke 7 bis 12 bzw. Rollen weisen ein weibliches Gelenkelement 13.1 bis 13.4 auf. In dieses weibliche Gelenkelement 13.1 bis 13.4 greift jeweils eine nicht dargestelltes männliches Gelenkelement, das in der zweiten Zangenhälfte 3 bzw. der anderen Seite des Zwischenstücks 9 bzw. 10 angeordnet ist.

Die Zange 1 umfasst vier Spülkanäle 14, 15, 16 und 23.

Im Folgenden wird beispielhaft die Anordnung des Spülkanals 23 beispielhaft an dem zweiten Gelenk 8 beschrieben. Die Spülkanäle 14, 15 und 16 sind an den entsprechenden Gelenken analog angeordnet.

Der Spülkanal 23 ist so an dem Gelenk 8 angeordnet, dass eine Innenfläche 17 (gestrichelt dargestellt) des weiblichen Elements 13.4 mit einer Oberfläche 18 der Zange 1 verbunden wird.

In einem nicht gezeigten Ausführungsbeispiel kann ein anderer Spülkanal so an einem anderen Gelenk angeordnet sein, dass eine andere Innenfläche des männlichen Funktionselements mit einer anderen Oberfläche der Zange verbunden sein.

Des Weiteren weisen die Zwischenstücke 9 und 10 jeweils an ihren zueinander zugewandten Innenseiten jeweils zwei Ausnehmungen 19 und 20 bzw. 21 und 22 auf. Die Ausnehmung 19 ist an der ersten Zangenhälfte 2 zwischen dem Gelenk 8 und dem Knickgelenk 11 vorgesehen. Die zweite Ausnehmung 20 ist an der ersten Zangenhälfte 2 zwischen den Gelenken 7 und dem Knickgelenk 11 vorgesehen.

Analog ist die Ausnehmung 21 an der zweiten Zangenhälfte 3 zwischen dem Gelenk 8 und dem Knickgelenk 12 vorgesehen. Die Ausnehmung 22 ist an der zweiten Zangenhälfte 3 zwischen dem Knickgelenk 12 und dem Gelenk 7 vorgesehen.

Die Funktionsweise der vorliegenden Erfindung ist folgende:

Nach Gebrauch kann die Zange 1 durch Ansetzen einer geeigneten Spülpistole bzw. mit einem geeigneten Aufsatz an einen der Spülkanäle 14, 15, 16 oder 23 gereinigt werden. Dabei dringt Spülflüssigkeit in das entsprechende Gelenk und zwischen die Zangenhälften 2 und 3 ein und reinigt den Zwischenraum bzw. die Flächen zwischen den weiblichen und männlichen Gelenkelementen.

Die Ausnehmungen 21 und 19 bzw. 22 und 20 sind so vorgesehen, dass bei unbelasteter Zange 1 die Zwischenstücke 9 und 10 zumindest teilweise voneinander beabstandet sind. Dadurch kann in diesem Bereich durch die Spülkanäle eingebrachte Spülflüssigkeit besonders gut austreten.

Ein weiterer Vorteil ist, die Zange 1 in einer Spülmaschine besser gereinigt werden kann, da sich die Innenflächen an den Ausnehmungen 19 und 21 bzw. 22 und 20 nicht berühren.

### Bezugszeichenliste

| | | | | | |
|---|---|---|---|---|---|
| 1 | Zange | 34 | | 67 | |
| 2 | erste Zangehälfte | 35 | | 68 | |
| 3 | zweite Zangenhälfte | 36 | | 69 | |
| 4 | Zangenmaul | 37 | | 70 | |
| 5 | Branche | 38 | | 71 | |
| 6 | Branche | 39 | | 72 | |
| 7 | Erstes Gelenk | 40 | | 73 | |
| 8 | Zweites Gelenk | 41 | | 74 | |
| 9 | Zwischenstück | 42 | | 75 | |
| 10 | Zwischenstück | 43 | | 76 | |
| 11 | Knickgelenk | 44 | | 77 | |
| 12 | Knickgelenk | 45 | | 78 | |
| 13 | Weibliches Funktionselement | 46 | | 79 | |
| 14 | Spülkanal | 47 | | | |
| 15 | Spülkanal | 48 | | | |
| 16 | Spülkanal | 49 | | | |
| 17 | Innenfläche | 50 | | | |
| 18 | Oberfläche | 51 | | | |
| 19 | Ausnehmung | 52 | | | |
| 20 | Ausnehmung | 53 | | | |
| 21 | Ausnehmung | 54 | | | |
| 22 | Ausnehmung | 55 | | | |
| 23 | Spülkanal | 56 | | | |
| 24 | | 57 | | | |
| 25 | | 58 | | | |
| 26 | | 59 | | | |
| 27 | | 60 | | | |
| 28 | | 61 | | | |
| 29 | | 62 | | | |
| 30 | | 63 | | | |
| 31 | | 64 | | | |
| 32 | | 65 | | | |
| 33 | | 66 | | | |

## Patentansprüche

1. Zange (1), insbesondere für chirurgische Zwecke, umfassend
eine erste Zangenhälfte (2) und
eine zweite Zangenhälfte (3), wobei die Zangenhälften (2, 3) über zumindest ein Gelenk (7) miteinander verbunden sind; und wobei
zumindest eine der Zangenhälften (2, 3) zumindest einen Spülkanal (15) aufweist; **dadurch gekennzeichnet; dass** der Spülkanal (15) so angeordnet ist, dass er eine Oberfläche (18) der Zangenhälfte (2) mit einer Innenfläche (17) eines Funktionselements (13.1) des Gelenks (7) verbindet.

2. Zange nach Anspruch 1, **dadurch gekennzeichnet, dass** die Funktionselemente des Gelenks (7) ein Spiel zueinander aufweisen.

3. Zange nach Anspruch 1 oder 2, **dadurch gekennzeichnet, dass** der Spülkanal (15) so angeordnet ist, dass er ein weibliches Funktionselement (13.1) des Gelenks (7) mit eine Oberfläche (18) der Zangenhälfte (2) verbindet.

4. Zange nach einem der Ansprüche 1 bis 3, **dadurch gekennzeichnet, dass** der Spülkanal (15) eine Spüldüse aufweist.

5. Zange nach einem der Ansprüche 1 bis 4, **dadurch gekennzeichnet, dass** der Spülkanal (15) geeignet ist, eine Spüldüse aufzunehmen.

6. Zange nach einem der Ansprüche 1 bis 5, **dadurch gekennzeichnet, dass** zumindest eine der Zangenhälften (2, 3), insbesondere zwischen einem Maulteil (4) und einer Branche (5, 6), zumindest eine Ausnehmung (19,10, 21, 22) aufweist, so dass die Zangenhälften (2, 3) in einem unbelasteten Zustand der Zange (1) zwischen dem Maulteil (4) und den Branchen (5, 6) zumindest teilweise voneinander beabstandet sind.

7. Zange nach einem der Ansprüche 1 bis 7, **dadurch gekennzeichnet, dass** die Zange (1) eine Mehrzahl von Gelenken (7, 8, 11, 12) aufweist, wobei zumindest eine Ausnehmung (10, 20, 21, 22) an einem die Gelenke verbindenden Zwischenstück (9, 10) vorgesehen ist.

8. Verwendung einer Spülpistole zum Spülen eines Spülkanals (15) einer Zange (1) einem nach einem der Ansprüche 1 bis 7.

## Claims

1. Forceps (1), in particular for surgical purposes, comprising
a first forceps half (2) and
a second forceps half (3), wherein the forceps halves (2, 3) are connected to one another via at least one articulation (7); and wherein
at least one of the forceps halves (2, 3) has at least one flushing channel (15);
**characterised in that** the flushing channel (15) is arranged in such a manner that it connects an upper surface (18) of the forceps half (2) to an inner surface (17) of a functional element (13.1) of the articulation (7).

2. Forceps according to claim 1, **characterised in that** the functional elements of the articulation (7) have clearance in relation to one another.

3. Forceps according to claim 1 or 2, **characterised in that** the flushing channel (15) is arranged in such a manner that it connects a female functional element (13.1) of the articulation (7) to an upper surface (18) of the forceps half (2).

4. Forceps according to any one of claims 1 to 3, **characterised in that** the flushing channel (15) has a flushing nozzle.

5. Forceps according to any one of claims 1 to 4, **characterised in that** the flushing channel (15) is suitable for receiving a flushing nozzle.

6. Forceps according to any one of claims 1 to 5, **characterised in that** at least one of the forceps halves (2, 3) has, in particular between a mouth part (4) and a branch (5, 6), at least one recess (19, 10, 21, 22), so that the forceps halves (2, 3) are in an unloaded state of the forceps (1) at least partially spaced apart from one another between the jaw part (4) and the branches (5, 6).

7. Forceps according to any one of claims 1 to 7, **characterised in that** the forceps (1) have a plurality of articulations (7, 8, 11, 12), wherein at least one recess (10, 20, 21, 22) is provided on an intermediate piece (9, 10) connecting the articulations.

8. Use of a flushing gun for flushing a flushing channel (15) of forceps (1) according to any one of claims 1 to 7.

## Revendications

1. Pince (1), en particulier à des fins chirurgicales, comprenant
une première demi-pince (2) et
une deuxième demi-pince (3), les demi-pinces (2, 3) étant reliées l'une à l'autre par l'intermédiaire d'au moins une articulation (7), et
au moins l'une des demi-pinces (2, 3) présentant au moins un canal de rinçage (15);
**caractérisée par le fait que** le canal de rinçage (15) est aménagé de sorte qu'il relie une surface (18) de la demi-pince (2) avec une surface intérieure (17) d'un élément fonctionnel (13.1) de l'articulation (7).

2. Pince selon la revendication 1, **caractérisée par le fait que** les éléments fonctionnels de l'articulation (7) présentent un jeu entre eux.

3. Pince selon la revendication 1 ou 2, **caractérisée par le fait que** le canal de rinçage (15) est aménagé de sorte qu'il relie un élément fonctionnel femelle (13.1) de l'articulation (7) avec une surface (18) de la demi-pince (2).

4. Pince selon l'une des revendications 1 à 3, **caractérisée par le fait que** le canal de rinçage (15) présente une tuyère de rinçage.

5. Pince selon l'une des revendications 1 à 4, **caractérisée par le fait que** le canal de rinçage (15) est adapté pour recevoir une tuyère de rinçage.

6. Pince selon l'une des revendications 1 à 5, **caractérisée par le fait qu'**au moins l'une des demi-pinces (2, 3) présente, en particulier entre une partie de mâchoire (4) et une branche (5, 6), au moins un évidement (19, 10, 21, 22) de sorte que les demi-pinces (2, 3) soient, dans un état non sollicité de la pince (1) au moins partiellement distantes l'une de l'autre entre la partie de mâchoire (4) et les branches (5, 6).

7. Pince selon l'une des revendications 1 à 7, **caractérisée par le fait que** la pince (1) présente une pluralité d'articulations (7, 8, 11, 12), au moins un évidement (10, 20, 21, 22) étant prévu dans une pièce intermédiaire (9, 10) reliant les articulations.

8. Utilisation d'un pistolet de rinçage pour rincer un canal de rinçage (15) d'une pince (1) selon l' une des revendications 1 à 7.
